# EUROPEAN PATENT APPLICATION

(11) **EP 4 166 645 A1**
(43) Date of publication of application: **19.04.2023**
(21) Application number: 21822211.5
(22) Date of filing: 09.06.2021
(51) Int. Cl.: C12M 3/00, C08F 297/02, C09D 7/20, C09D 133/14, C09D 153/00, C12N 5/071

(54) **BIOCOMPATIBLE COATING FILM CONTAINING BLOCK COPOLYMER**

(30) Priority: 12.06.2020 JP 2020102646
(71) Applicant: Nissan Chemical Corporation, Tokyo 103-6119 (JP)
(72) Inventor: HIROI Miya, Funabashi-shi, Chiba 274-0052 (JP); TADOKORO Shinsuke, Funabashi-shi, Chiba 274-8507 (JP); KOZAWA Masami, Funabashi-shi, Chiba 274-8507 (JP); HIROI Yoshiomi, Funabashi-shi, Chiba 274-0052 (JP)
(74) Representative: dompatent von Kreisler Selting Werner - Partnerschaft von Patent- und Rechtsanwälten mbB
(86) International application number: PCT/JP2021/021861
(87) International publication number: WO 2021/251417

(57) **Abstract**

This is to provide a composition for forming a coating film having compatibility with a biological substance which comprises a block copolymer having a specific structure, a coating film using the same, a substrate for cell culture using the same, a method for producing the substrate for cell culture, and a method for producing a cell aggregate.

This is the composition for forming a coating film having compatibility with a biological substance which comprises a block copolymer having unit structures represented by the formula (1) and the formula (2): 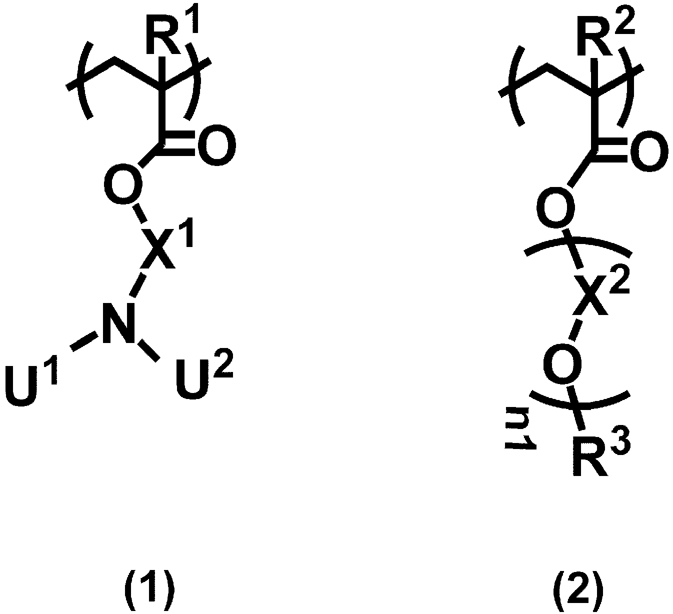 (wherein R¹ to R³, U¹ and U² each independently represent a hydrogen atom or an alkyl group having 1 to 5 carbon atoms, X¹ and X² represent an alkylene group having 1 to 5 carbon atoms, and n1 represents an integer of 1 to 10), and a solvent.

## Description

### TECHNICAL FIELD

The present invention relates to a composition for forming a coating film having compatibility with a biological substance containing a block copolymer, a coating film using the same, a substrate for cell culture using the same, a method for producing a substrate for cell culture, and a method for producing a cell aggregate.

### BACKGROUND ART

Various coating materials having an ability to suppress adhesion of biological substances have been proposed for suppressing adhesion of biological substances of devices for medical use such as artificial dialyzers, artificial organs, medical devices, etc., and various kinds of devices for medical use such as cell culture containers, substrates for cell culture, etc.

In Patent Document 1, there is disclosed an ion complex material having an ability to suppress adhesion of a biological substance. In Patent Document 2, there are disclosed a method for producing a polymer used as a base film for cell culture and a cell culture container.

### PRIOE ART DOCUMENTS

### PATENT DOCUMENTS

Patent Document 1: WO 2014/196650
Patent Document 2: WO 2020/040247

### SUMMARY OF THE INVENTION

### PROBLEMS TO BE SOLVED BY THE INVENTION

An object of the present invention is to provide a composition for forming a coating film having compatibility with a biological substance containing a block copolymer having a specific structure, a coating film using the same, a substrate for cell culture using the same, a method for producing the substrate for cell culture, and a method for producing a cell aggregate(s).

The present inventors have found that a coating film containing a block copolymer having a specific structure is particularly excellent in n ability to suppress adhesion of a protein, and as a coating film of a substrate for cell culture, it has a peculiar property that cells are attracted but not adhered, and thus, it exerts an excellent effect particularly in the production of a cell aggregate(s), whereby they have accomplished the present invention.

### MEANS TO SOLVE THE PROBLEMS

The present invention is as follows.
[1] A composition for forming a coating film having compatibility with a biological substance which comprises a block copolymer having unit structures represented by the formula (1) and the formula (2): 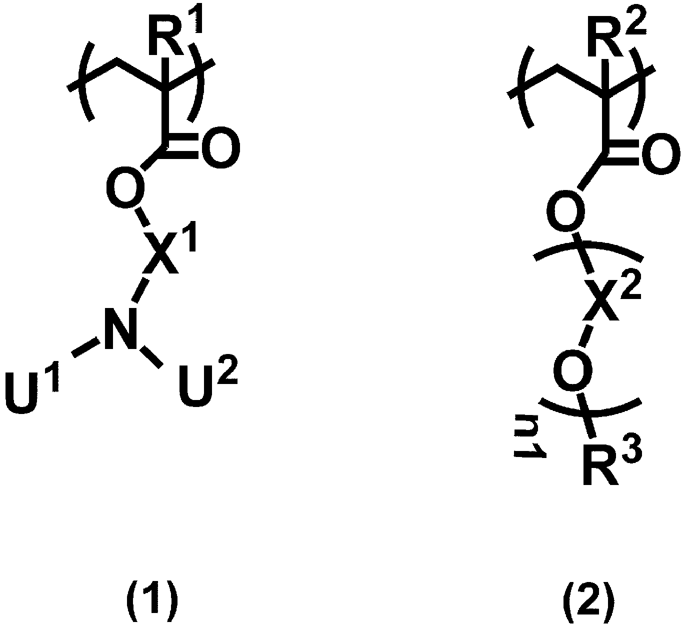 (wherein R¹ to R³, U¹ and U² each independently represent a hydrogen atom or an alkyl group having 1 to 5 carbon atoms, X¹ and X² each independently represent an alkylene group having 1 to 5 carbon atoms, and n1 represents an integer of 1 to 10), and a solvent.
[2] The composition described in the above-mentioned [1], wherein the solvent contains water or an alcohol.
[3] The composition described in the above-mentioned [1] or [2], wherein the compatibility with a biological substance is an ability to suppress adhesion of a protein.
[4] The composition described in the above-mentioned [1] or [2], wherein the compatibility with a biological substance is for forming a base film for cell culture.
[5] The composition described in the above-mentioned [4], which is used for forming a base film for cell culture to obtain a cell aggregate by adhering and then peeling cells.
[6] A coating film having compatibility with a biological substance which is a coating film of the composition for forming a coating film having compatibility with a biological substance described in any one of the above-mentioned [1] to [5].
[7] A substrate for cell culture, which comprises the coating film having compatibility with a biological substance described in the above-mentioned [6] at least a part of a surface of a substrate having an ability to suppress adhesion of a biological substance.
[8] The substrate for cell culture described in the above-mentioned [7], wherein the substrate having an ability to suppress adhesion of a biological substance is a substrate provided with a coating film containing a copolymer which contains a recurring unit containing a group represented by the following formula (a) and a recurring unit containing a group represented by the following formula (b): 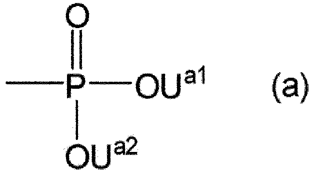 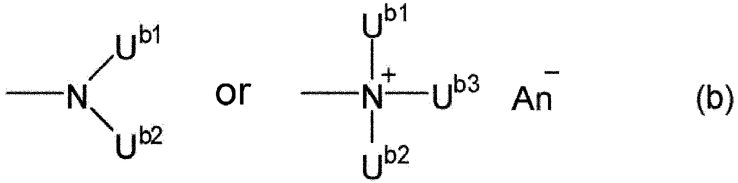 (wherein
   U^{a1}, U^{a2}, U^{b1}, U^{b2} and U^{b3} each independently represent a hydrogen atom or an alkyl group having 1 to 5 carbon atoms, and An⁻ represents an anion selected from the group consisting of a halide ion, an inorganic acid ion, a hydroxide ion and an isothiocyanate ion) on at least a part of a surface thereof.
[9] A method for producing a substrate for cell culture which comprises a step of applying a composition for forming a coating film having compatibility with a biological substance containing a block copolymer having unit structures represented by the formula (1) and the formula (2): 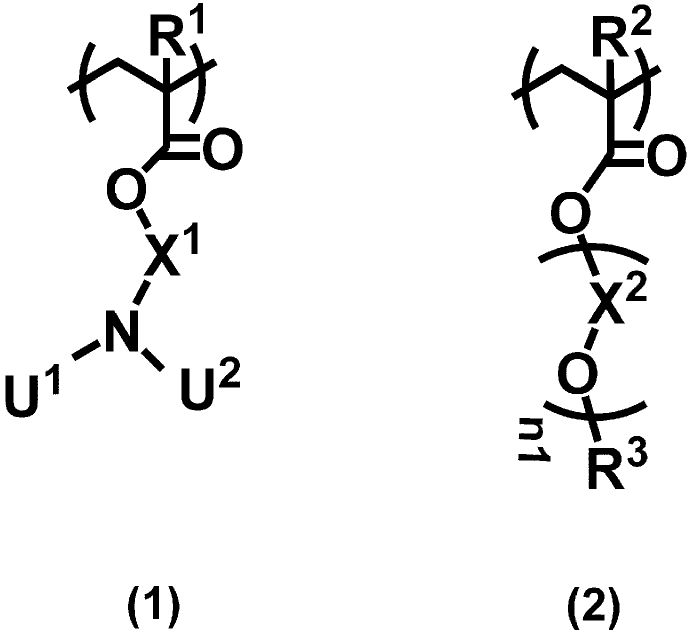 (wherein R¹ to R³, U¹ and U² each independently represent a hydrogen atom or an alkyl group having 1 to 5 carbon atoms, X¹ and X² each independently represent an alkylene group having 1 to 5 carbon atoms, and n1 represents an integer of 1 to 10), and a solvent to a substrate having an ability to suppress adhesion of a biological substance, and then, a step of drying the same.
[10] A method for producing a cell aggregate which comprises a step of providing a base film for cell culture comprising a block copolymer having unit structures represented by the formula (1) and the formula (2): 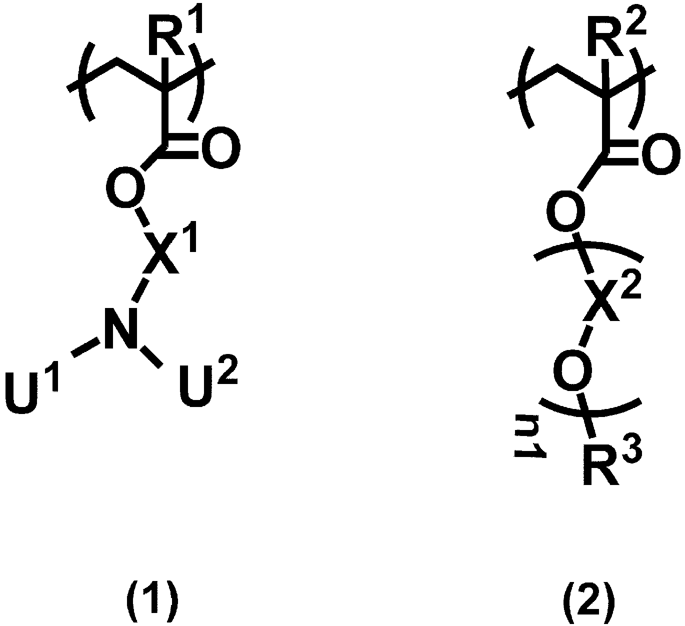 (wherein R¹ to R³, U¹ and U² each independently represent a hydrogen atom or an alkyl group having 1 to 5 carbon atoms, X¹ and X² each independently represent an alkylene group having 1 to 5 carbon atoms, and n1 represents an integer of 1 to 10) onto at least a part of a surface of a substrate, and a step of seeding cells on the base film.

### EFFECTS OF THE INVENTION

The block copolymer of the present invention contains a hydrophobic portion (for example, the block having the unit structure represented by the formula (1)) and a hydrophilic portion (for example, the block having the unit structure represented by the formula (2)), and when this is formed as a coating film onto a substrate, it is excellent in an ability to suppress adhesion of a protein, and further has a peculiar property that cells are attracted but not adhered. By taking the structure that the hydrophobic portion adheres to the substrate and the hydrophilic portion is exposed on the surface, it is presumed that the surface of the substrate exhibits hydrophilicity, and thus, it has peculiar characteristics for the above-mentioned cells.

### BRIEF DESCRIPTION OF THE DRAWINGS

Fig. 1 is a schematic drawing of a flow reactor (reaction device) used in Synthetic Examples 1 to 5.
Fig. 2 is a ¹H-NMR chart of the polymer obtained in Synthetic Example 1.
Fig. 3 is a ¹H-NMR chart of the polymer obtained in Synthetic Example 4.
Fig. 4 is a photograph microscopically observed the state of cell adhesion in a petri dish (schale) coated with each composition for forming a coating film of Preparation Examples 6 to 9 in Test Example 4.
Fig. 5 is a photograph microscopically observed the state of formation of cell aggregation in a petri dish coated with each composition for forming a coating film of Preparation Examples 6 to 9 in Test Example 4.

### EMBODIMENTS TO CARRY OUT THE INVENTION

### <Composition for forming coating film having compatibility with biological substance>

The composition for forming a coating film having compatibility with a biological substance of the present invention is a composition for forming a coating film having compatibility with a biological substance which comprises a block copolymer having unit structures represented by the formula (1) and the formula (2): 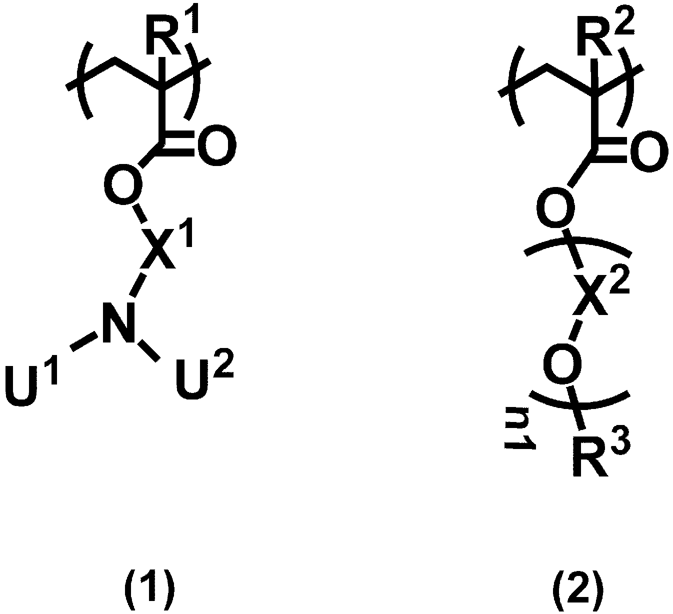 (wherein R¹ to R³, U¹ and U² each independently represent a hydrogen atom or an alkyl group having 1 to 5 carbon atoms, X¹ and X² each independently represent an alkylene group having 1 to 5 carbon atoms, and n1 represents an integer of 1 to 10), and a solvent.

As the above-mentioned alkyl group having 1 to 5 carbon atoms, there may be mentioned a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group or a 1-ethylpropyl group. R¹ to R³, U¹ and U² each independently and preferably selected from a hydrogen atom, a methyl group or an ethyl group.

As the above-mentioned alkylene group having 1 to 5 carbon atoms, there may be mentioned a methylene group, an ethylene group, a propylene group, a trimethylene group, a tetramethylene group, a 1-methylpropylene group, a 2-methylpropylene group, a dimethylethylene group, an ethylethylene group, a pentamethylene group, a 1-methyl-tetramethylene group, a 2-methyl-tetramethylene group, a 1,1-dimethyl-trimethylene group, a 1,2-dimethyl-trimethylene group, a 2,2-dimethyl-trimethylene group and a 1-ethyl-trimethylene group. As the above-mentioned X¹ and X², they are preferably selected from a methylene group, an ethylene group and a propylene group.

As the above-mentioned n1, an integer of 1 to 6 is preferable, and an integer of 2 to 4 is more preferable.

The unit structure represented by the above-mentioned the formula (1) is preferably derived from compounds represented by the following formula (1-1) and the formula (2-1): 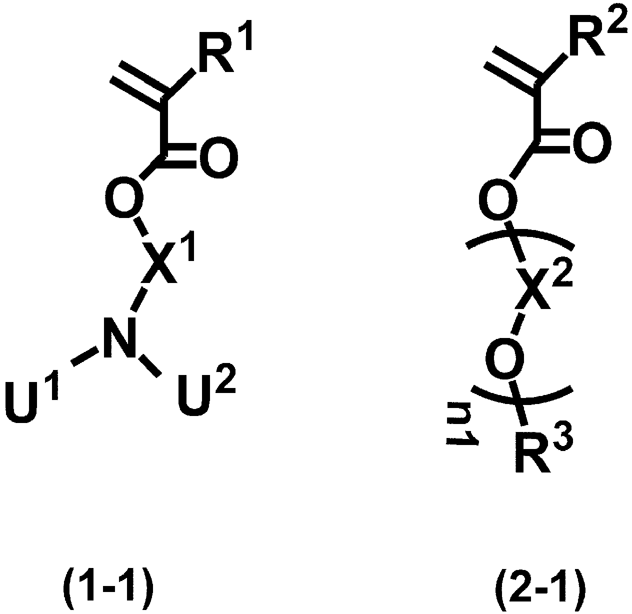 In the formulae, the meanings and preferable embodiments of R¹ to R³, U¹ and U², X¹ and X², and n1 are the same as defined above.

Specific example of the monomer represented by the formula (1-1) may be mentioned 2-dimethylaminoethyl methacrylate, 2-diethylaminoethyl methacrylate, 2-dipropylaminoethyl methacrylate, etc., and it is preferable to use 2-dimethylaminoethyl methacrylate and 2-diethylaminoethyl methacrylate.

Specific example of the monomer represented by the formula (2-1) may be mentioned diethylene glycol monomethyl ether methacrylate, dipropylene glycol monomethyl ether methacrylate, etc., and it is preferable to use diethylene glycol monomethyl ether methacrylate.

The block copolymer of the present invention preferably contain two kinds of blocks polymerized from each one kind of the compounds which derive the units of the above-mentioned the formula (1) and the formula (2), or may contain three kinds or more blocks polymerized from one or both of them are two or more kinds of compounds.

It may further polymerize a compound which derives the recurring unit of the third component represented by a formula other than the above-mentioned the formula (1) and the formula (2).

The respective molar ratio of the unit structures represented by the formula (1) and the formula (2) is preferably the formula (1): the formula (2) =20 to 90 : 80 to 10, and preferably 40 to 70 : 60 to 30.

The molar ratio of the unit structures represented by the formula (1) and the formula (2) based on the whole block copolymer is not particularly limited as long as the compatibility with a biological substance is not impaired, and, for example, it is 80 mol% or more, 90 mol% or more, and 100 mol%.

The above-mentioned block copolymer can be synthesized by a known method, and may be produced, for example, using a flow reactor equipped with a specific mixer described in WO 2017/135398.

The block copolymer of the present invention is a material in which a plural kinds of polymers (blocks) which are chemically different and covalently bonded to each other are bonded. The block copolymer used in the present invention contains an organic polymer chain (A) which contains an organic monomer (a) as a unit structure, and a polymer chain (B) which contains a monomer (b) different from the organic monomer (a) as a unit structure and which binds to the organic polymer chain (A). As the specific embodiment of the block copolymer used in the present invention, there may be mentioned a polymer which contains the polymer (block) comprising the recurring unit represented by the formula (1) and the polymer (block) comprising the recurring unit represented by the formula (2), and the both blocks are covalently bonded to each other, etc.

A solid content of the composition for forming a coating film of the present invention can be made 0.01 to 50% by mass, or 0.1 to 20% by mass, or 0.1 to 10% by mass. The solid content is a remaining ratio in which the solvent is removed from the composition for forming a film.

A ratio of the block copolymer occupied in the solid content can be made 30 to 100% by mass, or 50 to 100% by mass, or 50 to 90% by mass, or 50 to 80% by mass.

A kind of the block existing in the block copolymer can be made 2 or 3 or more.

And a number of the blocks existing in the block copolymer can be made 2 or 3 or more.

By changing the polymer chain (B), for example, it is possible to use an adjacent polymer chain (C) containing a monomer (c) as a unit structure. As the block polymer, there are combinations of AB, ABAB, ABA, ABC, etc.

As one of the methods for synthesizing block copolymers, it can be obtained by living radical polymerization, living cationic polymerization or living anionic polymerization in which the polymerization process consists only of an initiation reaction and a growth reaction and is not accompanied by a side reaction that inactivates the growth end. The growth end can continue to maintain the growth activity reaction during the polymerization reaction. By eliminating chain transfer, a polymer (A) having a uniform length can be obtained. By adding a different monomer (b) using the growth end of the polymer (A), polymerization can proceed under the monomer (b) to form a block copolymer (AB).

The homopolymer A or B is a polymerizable compound having at least one polymerizable reactive group, a preferably a radically polymerizable reactive group (vinyl group or vinyl group-containing organic group).

A weight average molecular weight Mw of the block copolymer to be used in the present invention is preferably 1,000 to 1,000,000, 5,000 to 500,000, 5,000 to 100,000 or 5,000 to 50,000. If it is less than 1,000, there is a case where coatability to the substrate may be poor, while if it exceeds 1,000,000, there is a case where solubility in a solvent may be poor.

A polydispersity (Mw/Mn) of the block copolymer of the present invention is preferably 1.00 to 2.00, and particularly preferably 1.00 to 1.50.

A solvent contained in the composition for forming a coating film having compatibility with a biological substance of the present invention may be mentioned water, phosphate buffered physiological saline (PBS), alcohol or a mixed solvent in which two or more kinds thereof are combined. As the alcohol, there may be mentioned an alcohol having 2 to 6 carbon atoms, for example, ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (neopentyl alcohol), 2-methyl-1-propanol, 2-methyl-1-butanol, 2-methyl-2-butanol (t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol and cyclohexanol, and they may be used alone or a mixed solvent in combination thereof, but from the viewpoint of dissolving the block copolymer, it is preferably selected from water, PBS, ethanol and a mixed solvent in which two or more kinds thereof are combined.

In the present invention, as the biological substance, there may be mentioned a protein, a saccharide, a virus, a nucleic acid and a cell or a combination thereof.

The terms "having compatibility with a biological substance" mean that it has an ability to suppress adhesion of the above-mentioned biological substance (in particular, protein) and has no toxicity to the above-mentioned biological substance. Or else, it means that it can be used for forming a base film for cell culture, and particularly preferably for forming a base film for cell culture for obtaining a cell aggregate(s).

As the above-mentioned protein, there may be mentioned fibrinogen, bovine serum albumin (BSA), human albumin, various kinds of globulins, β-lipoprotein, various kinds of antibodies (IgG, IgA and IgM), peroxidase, various kinds of complements, various kinds of lectins, fibronectin, lysozyme, von Willebrand factor (vWF), serum γ-globulin, pepsin, ovalbumin, insulin, histone, ribonuclease, collagen and cytochrome c,
for example, as the saccharide, glucose, galactose, mannose, fructose, heparin and hyaluronic acid,
for example, as the nucleic acid, deoxyribonucleic acid (DNA) and ribonucleic acid (RNA), and
as the above-mentioned cells, fibroblast, bone marrow cells, B lymphocytes, T lymphocytes, neutrophils, red blood cells, platelets, macrophages, monocytes, bone cells, pericytes, dendritic cells, keratinocytes, fat cells, mesenchymal cells, epithelial cells, epidermal cells, endothelial cells, vascular endothelial cells, hepatic parenchymal cells, cartilage cells, cumulus cells, neural cells, glial cells, neurons, oligodendrocytes, microglia, astroglial cells, heart cells, esophagus cells, muscle cells (for example, smooth muscle cells or skeletal muscle cells), pancreatic beta cells, melanocytes, hematopoietic precursor cells, mononuclear cells, embryonic stem cells (ES cell), embryonic tumor cells, embryonic germline stem cells, induced pluripotent stem cells (iPS cell), neural stem cells, hematopoietic stem cells, mesenchymal stem cells, liver stem cells, pancreatic stem cells, muscle stem cells, germline stem cells, intestinal stem cells, cancer stem cells, hair follicle stem cells and various kinds of cell lines (for example, HCT116, Huh7, HEK293 (human embryonic kidney cell), HeLa (human cervical cancer cell lines), HepG2 (human liver cancer cell lines), UT7/TPO (human leukemia cell lines), CHO (Chinese hamster ovary cell lines), MDCK, MDBK, BHK, C-33A, HT-29, AE-1, 3D9, Ns0/1, Jurkat, NIH3T3, PC12, S2, Sf9, Sf21, High Five and Vero), etc.

The terms having an ability to suppress adhesion of a protein mean that, in the QCM-D measurement carried out by the method described in Examples, a mass (%) per a relative unit area ((a mass (ng/cm²) per a unit area of Example/(a mass (ng/cm²) per a unit area of no coating film)) when compared with no coating film is 50% or less, preferably 30% or less, and further preferably 20% or less; and
the terms having an ability to suppress adhesion of a cell mean that, a relative absorbance (WST O.D. 450 nm) (%) ((absorbance (WST O.D. 450 nm) of Example)/(absorbance (WST O.D. 450 nm) of Comparative Example)) when compared with no coating film by the fluorescence microscope carried out by the method described in WO 2016/093293 is 50% or less, preferably 30% or less, and further preferably 20% or less.

The above-mentioned compatibility with a biological substance is preferably an ability to suppress adhesion of a protein.

The above-mentioned compatibility with a biological substance is preferably for forming a base film for cell culture.

It is preferably for forming a base film for cell culture by adhering cells and then peeling the same to obtain a cell aggregate(s). Incidentally, the cell aggregate(s) refers to a structure formed as a result that the cells are aggregated, and its shape is not limited to a spherical shape and a ring shape, etc. As compared with the cell aggregate(s) prepared on a conventional cell low-adhesion plate by non-adhesive culture, there are merits in the points of size adjustment (any size of cell aggregate(s) can be produced) according to the regulation of the adhesion area, etc.

In addition to the above-mentioned block copolymer and the solvent, other substances may be added to the composition of the present invention, if necessary, in the range as long as the properties of the obtainable coating film is not impaired. As the other substances, there may be mentioned preservatives, surfactants, primers that enhances adhesion to a substrate, fungicides and saccharides, etc.

### <Coating film having compatibility with biological substance>

The coating film having compatibility with a biological substance of the present invention is a coating film of the above-mentioned composition for forming the coating film having compatibility with a biological substance, and typically, can be formed by applying the composition for forming a coating film according to the present invention onto a substrate and dried to form a coating film.

As the substrate for forming the coating film of the present invention, the same material as described in the following substrate for cell culture can be used.

For forming the coating film of the present invention, the above-mentioned composition for forming a coating film is coated onto at least a part of the surface of the substrate. As the applying method, it is not particularly limited, and usual applying method such as spin coating, dip coating, solvent cast method, etc., are used.

A drying step of the coating film according to the present invention is carried out under an atmosphere or vacuum, preferably at a temperature in the range of -200°C to 200°C. The coating film can be formed by drying, for example, at room temperature (10°C to 35°C, for example, 25°C), and for forming the coating film more quickly, it may be dried at, for example, 40°C to 100°C.

More preferable drying temperature is 10°C to 180°C, and more preferable drying temperature is 20°C to 150°C.

### <Substrate for cell culture, method for producing substrate for cell culture, substrate having an ability to suppress adhesion of a biological substance>

This is a substrate for cell culture, which comprises a coating film having compatibility with a biological substance at least a part of the surface of a substrate having an ability to suppress adhesion of a biological substance.

The substrate referred to in the present invention may be, in addition to a flat plate substrate having a plane surface, a container shape material (the so-called cell culture containers) with a structure having recesses such as wells, dishes, etc.

A substrate for cell culture can be produced by applying the above-mentioned composition for forming the coating film having compatibility with a biological substance onto the surface of the substrate and drying the same. Here, the "surface" refers to a surface in contact with the contents such as cells or cell culture medium, etc.

The substrate for cell culture of the present invention may also be provided with a coating film having compatibility with a biological substance as a plurality of spots onto a substrate having an ability to suppress adhesion of a biological substance. A shape of the spot is not particularly limited. It is, for example, a substantially circular or quadrangular shape, and it is preferably a substantially circular spot.

A ratio of the total area of the spots to the surface area of the substrate, a diameter of each spot and a distance between the spots may be appropriately selected from a predetermined range according to the kind of the cells or substrates to be used and the desired size of the cell aggregate(s), and the ratio of the total area of the spots to the surface area of the substrate is preferably 30% or more, 40% or more and 50% or more, and preferably 99% or less, the diameter of each spot is preferably 50 to 5,000 µm, and 300 to 3,000 µm, and the distance between the spots is preferably 30 to 1,000 µm, and 100 to 500 µm

In the substrate for cell culture of the present invention, an independent micro-sized region (spot) having compatibility with a biological substance can be formed onto the substrate having an ability to suppress adhesion of a biological substance by arranging them with such a high density and, preferably regularly, to form a plurality of cell aggregates with uniform size onto one substrate (container) at one time.

As the substrate (in particular, cell culture containers), there may be mentioned, for example, petri dishes or dishes such as petri dishes generally used for culture of cells, dishes for tissue culture, multi dishes, etc., flasks such as cell culture flasks, spinner flasks, etc., bags such as plastic bags, Teflon (Registered Trademark) bags, culture bags, etc., plates such as microplates, microwell plates, multi plates, multiwell plates, etc., a bottle such as chamber slides, tubes, trays, roller bottles, etc. It is preferably mentioned petri dishes or dishes, plates and trays.

Also, the material of the substrate may be mentioned, for example, glass, metal, a metal-containing compound or a semi metal-containing compound, activated charcoal or a resin. The metals may be mentioned typical metals: (alkali metals: Li, Na, K, Rb and Cs; alkaline earth metals: Ca, Sr, Ba and Ra), magnesium group elements: Be, Mg, Zn, Cd and Hg; aluminum group elements: Al, Ga and In; rare earth elements: Y, La, Ce, Pr, Nd, Sm and Eu; tin group elements: Ti, Zr, Sn, Hf, Pb and Th; iron group elements: Fe, Co and Ni; earth acid elements: V, Nb and Ta, chromium group elements: Cr, Mo, W and U; manganese group elements: Mn and Re; noble metals: Cu, Ag and Au; platinum group elements: Ru, Rh, Pd, Os, Ir and Pt, etc. The metal-containing compound or the semi metal-containing compound may be mentioned, for example, ceramics comprising a metal oxide as a basic component, which are a sintered body baked by a heat treatment at a high temperature, a semiconductor such as silicon, an inorganic solid material including a molded product of an inorganic compound such as a metal oxide or a semi-metal oxide (silicon oxide, alumina, etc.), a metal carbide or a semi-metal carbide, a metal nitride or a semi-metal nitride (silicon nitride, etc.), a metal boride or a semi-metal boride, etc., aluminum, nickel-titanium and stainless (SUS304, SUS316, SUS316L, etc.).

As the resin, it may be either of a natural resin or a derivative thereof, or a synthetic resin, as the natural resin, there may be mentioned cellulose, cellulose triacetate (CTA), nitrocellulose (NC), cellulose immobilized with dextran sulfate, etc., and as the synthetic resin, there may be preferably used polyacrylonitrile (PAN), polyester-based polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester, polypropylene (PP), polyvinylidene fluoride (PVDF), polyether sulfone (PES), polycarbonate (PC), polyvinyl chloride (PVC), polytetrafluoroethylene (PTFE), ultra-high molecular weight polyethylene (UHPE), polydimethylsiloxane (PDMS), acrylonitrile-butadiene-styrene resin (ABS) or Teflon (Registered Trademark). In the production of the substrate for cell culture of the present invention, it does not require a treatment at a high temperature at the time of applying the composition for forming a coating film so as to exist at least a part of the surface of the substrate, so that a resin having low heat resistance, etc., can be also applicable.

A material of the substrate may be one kind or a combination of two or more kinds. Among these materials, it is preferably glass, silicon, silicon oxide, polystyrene(PS), polypropylene(PP), polyether sulfone (PES), polyethylene terephthalate (PET), poly carbonate (PC), poly vinyl chloride (PVC), Teflon (Registered Trademark), cycloolefin polymer (COP), polydimethylsiloxane (PDMS) or stainless (SUS304, SUS316, SUS316L, etc.) alone, or a combination selected from these, and particularly preferably glass, polystyrene(PS), polypropylene(PP), stainless (SUS304, SUS316, SUS316L, etc.) or polydimethylsiloxane (PDMS).

As a method for applying the composition for forming a coating film of the present invention, in addition to a spin coating, dip coating, solvent casting method, etc., there may be employed, for example, an inkjet method, a screen printing method, a slit coating method, a roll-to-roll method, etc., and preferably carried out by a printing technique such as the inkjet method or the screen printing method, etc.

As another applying method, for example, there may be employed a method in which the container is immersed in the above-mentioned composition for forming a coating film, the composition for forming a coating film is added to the container and allowed to stand for a predetermined time, or the composition for forming a coating film is coated onto the surface of the container or the substrate, etc., and in the container, as one embodiment, in the case of cell culture containers, it is carried out by the method in which the composition for forming a coating film is added to the container and allowed to stand for a predetermined time. Alternatively, when the coating film is a spot, for example, it is carried out by the method in which a substrate in which a non-forming portion(s) of the spots is/are occasionally protected is immersed in the above-mentioned composition for forming a coating film, or the composition for forming a coating film is added to the substrate (container) in which a non-forming portion(s) of the spots is/are occasionally protected, and allowed to stand for a predetermined time, etc. Addition can be carried out by adding, for example, 0.5 to 1-fold amount of the composition for forming a coating film based on the whole volume of the container using a syringe, etc. Allowing to stand can be practiced by optionally selecting a time and temperature depending on the material of the container or the substrate, the kind of the base film forming agent for cell culture, and is practiced, for example, 1 minute to 24 hours, preferably 5 minutes to 3 hours at 10 to 80°C. According to the procedure, a cell culture container having the base film for cell culture at least a part of the surface of the container, preferably over the entire surface, can be produced.

Also, the coating film on the surface of the container or the substrate obtained by such a method can be used as a cell culture container, after the step of contacting at least a part of the surface of the above-mentioned container or substrate, preferably after the step of adding the composition for forming a coating film and allowing to stand for a predetermined time, without subjecting to a drying step and as such, or after washing using water or a medium (for example, water, buffer, culture medium, etc.) of the sample applied to cell culture.

That is, it can be used as a cell culture container after the step of contacting at least a part of the surface of the above-mentioned container or substrate, preferably after the step of adding the composition for forming a coating film and allowing to stand for a predetermined time, within 48 hours, preferably within 24 hours, further preferably within 12 hours, further preferably within 6 hours, further preferably within 3 hours, further preferably within 1 hour without subjecting to the drying step and as such, or after washing with water or a medium (for example, water, buffer, culture medium, etc., particularly preferably medium (for example, BME medium (Eagle's basal medium), DMEM medium (Dulbecco's modified Eagle's medium)) of a sample applied to cell culture.

The container may be applied to a drying step. The drying step is carried out under an atmosphere or vacuum, preferably at a temperature in the range of -200°C to 200°C. By the drying step, the solvent in the above-mentioned base film forming agent is removed, whereby it is completely adhered to the substrate.

The coating film can be formed by drying, for example, at room temperature (10°C to 35°C, preferably 20°C to 30°C, for example, 25°C), and may be dried, for example, at 40°C to 100°C to form a base film more rapidly. More preferable drying temperature is 10°C to 180°C, and more preferable drying temperature is 20°C to 150°C.

The coating film of the present invention is produced by the simple and easy step as above.

Also, in order to eliminate impurities, unreacted monomers, etc., remained on the coating film, a step of washing with at least one kind of a solvent(s) selected from water and an aqueous solution containing an electrolyte(s) may be carried out. For washing, running water washing or ultrasonic wave washing, etc., is desirable. The above-mentioned water and aqueous solution containing an electrolyte(s) may be heated in the range of, for example, 40°C to 95°C. The aqueous solution containing an electrolyte(s) is preferably PBS, physiological saline (containing only sodium chloride), Dulbecco's phosphate buffered physiological saline, tris buffered physiological saline, HEPES buffered physiological saline and Veronal buffered physiological saline, and PBS is particularly preferable. After fixing, the coating film does not dissolve out and firmly fixed to the substrate even when it is washed with water, PBS and alcohol, etc.

A film thickness of the coating film of the present invention has a maximum film thickness and a minimum film thickness in the range of 1 to 1,000 nm, preferably in the range of 5 to 500 nm, 10 to 300 nm, 10 to 200 nm, 10 to 100 nm, or 10 to 50 nm.

### <Substrate having ability to suppress adhesion of biological substances>

As the substrate having an ability to suppress adhesion of a biological substance of the present invention, a known material such as a commercially available product, etc., can be used, and it is preferable to provide a coating film containing a copolymer which contains a recurring unit containing the group represented by the following formula (a) and a recurring unit containing the group represented by the following formula (b): 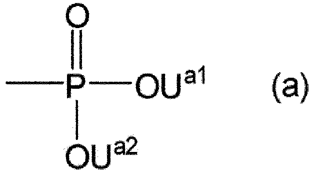 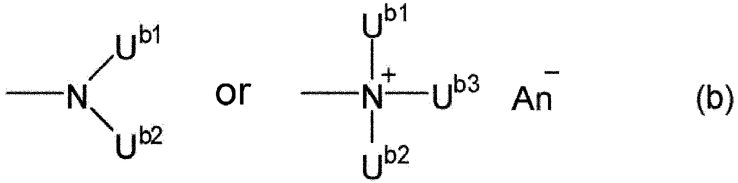 (wherein
U^{a1}, U^{a2}, U^{b1}, U^{b2} and U^{b3} each independently represent a hydrogen atom or an alkyl group having 1 to 5 carbon atoms, and An⁻ represents an anion selected from the group consisting of a halide ion, an inorganic acid ion, a hydroxide ion and an isothiocyanate ion) described in WO 2014/196650 at least a part of the surface of the substrate.

It is preferable that the above-mentioned copolymer contains recurring units of the following formula (a1) and the formula (b1): 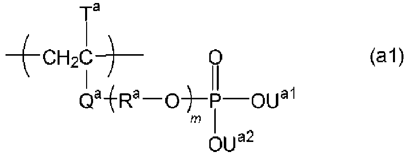 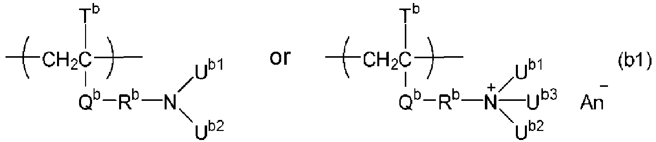 (wherein,
T^{a}, T^{b}, U^{a1}, U^{a2}, U^{b1}, U^{b2} and U^{b3} each independently represent a hydrogen atom or an alkyl group having 1 to 5 carbon atoms, Q^{a} and Q^{b} each independently represent a single bond, an ester bond or an amide bond, R^{a} and R^{b} each independently represent an alkylene group having 1 to 10 carbon atoms which may be substituted by a halogen atom(s), An⁻ represents an anion selected from the group consisting of a halide ion, an inorganic acid ion, a hydroxide ion and an isothiocyanate ion, and m represents an integer of 0 to 6).

The above-mentioned alkyl group having 1 to 5 carbon atoms is as that mentioned in the formulae (1) and (2). The above-mentioned alkylene group having 1 to 10 carbon atoms which may be substituted by a halogen atom(s) means that it is an alkylene group having 1 to 10 carbon atoms, or an alkylene group having 1 to 10 carbon atoms substituted by one or more halogen atoms, here, as the alkylene group having 1 to 10 carbon atoms, there may be mentioned, in addition to the examples mentioned in the alkylene group having 1 to 5 carbon atoms of the formulae (1) and (2), a hexamethylene group, an octamethylene group and a decamethylene group, etc. And the above-mentioned linear or branched alkylene group having 1 to10 carbon atoms substituted by one or more halogen atom(s) means those in which one or more optional hydrogen atom(s) of the above-mentioned alkylene group is/are substituted by a halogen atom(s), here, the above-mentioned halogen atom(s) may be mentioned a fluorine atom, a chlorine atom, a bromine atom and an iodine atom.

Further, the above-mentioned terms "halide ion" mean a fluoride ion, a chloride ion, a bromide ion or an iodide ion, and the above-mentioned inorganic acid ion means a carbonate ion, a sulfate ion, a phosphate ion, a hydrogen phosphate ion, a dihydrogen phosphate ion, a nitrate ion, a perchlorate ion or a borate ion.

Details of the coating film using the above-mentioned copolymer are based on the contents described in WO 2014/196650.

As the other coating film having an ability to suppress adhesion of a biological substance, a material in which an ethylenically unsaturated monomer, or a polysaccharide or a derivative thereof are copolymerized may be used. Examples of the ethylenically unsaturated monomer may be mentioned one kind or two or more kinds of ethylenically unsaturated monomers selected from the group consisting of (meth)acrylic acid and an ester thereof; vinyl acetate; vinyl pyrrolidone; ethylene; vinyl alcohol; and their hydrophilic functional derivatives. Examples of the polysaccharide or a derivative thereof may be mentioned cellulose-based polymers such as hydroxyalkyl cellulose (for example, hydroxyethyl cellulose or hydroxypropyl cellulose), etc., starch, dextran and curdlan.

The hydrophilic functional derivatives refer to an ethylenically unsaturated monomer having a hydrophilic functional group or structure. Examples of the hydrophilic functional group or structure may be mentioned a betaine structure; an amide structure; an alkylene glycol residue; an amino group; and a sulfinyl group, etc.

The betaine structure means a monovalent or divalent group of a compound having an amphoteric center of a quaternary ammonium type cation structure and an acidic anion structure, and there may be mentioned, for example, a phosphorylcholine group: 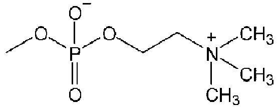 Examples of the ethylenically unsaturated monomers having such a structure may be mentioned 2-methacryloyloxyethyl phosphorylcholine (MPC), etc.

The amide structure means a group represented by the following formula: 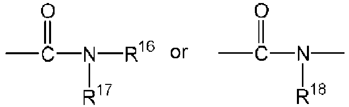 [here, R¹⁶, R¹⁷ and R¹⁸ each independently represent a hydrogen atom or organic group (for example, a methyl group, a hydroxymethyl group or hydroxyethyl group, etc.)]. Examples of the ethylenically unsaturated monomer having such a structure may be mentioned (meth)acrylamide, N-(hydroxymethyl)(meth)acrylamide, etc. Further, the monomer or polymer having such a structure is disclosed in, for example, JP 2010-169604A, etc.

The alkylene glycol residue means an alkyleneoxy group (-Alk-O-) which remains after the hydroxyl group of one terminal end or both terminal ends of the alkylene glycol (HO-Alk-OH; here, Alk is an alkylene group having 1 to 10 carbon atoms) undergoes a condensation reaction with another compound, and it includes a poly(alkyleneoxy) group in which an alkyleneoxy unit is repeated. Examples of the ethylenically unsaturated monomer having such a structure may be mentioned 2-hydroxyethyl (meth)acrylate, methoxypolyethylene glycol (meth)acrylate, etc. Further, the monomer or polymer having such a structure is disclosed in, for example, JP 2008-533489A, etc.

The amino group means a group represented by the formula: -NH₂, -NHR¹⁹ or -NR²⁰R²¹ [here, R¹⁹, R²⁰ and R²¹ are each independently an organic group (for example, an alkyl group having 1 to 5 carbon atoms, etc.)]. In the amino group in the present invention, a quaternized or chlorinated amino group is included. Examples of the ethylenically unsaturated monomer having such a structure may be mentioned dimethylaminoethyl (meth)acrylate, 2-(t-butylamino)ethyl (meth)acrylate, methacryloylcholine chloride, etc.

The sulfinyl group means a group represented by the following formula: 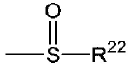 [here, R²² is an organic group (for example, an organic group having 1 to 10 carbon atoms, a preferably an alkyl group having 1 to 10 carbon atoms and having one or more hydroxy group(s), etc.)].
As a polymer having such a structure, there may be mentioned a copolymer disclosed in JP 2014-48278A.

### <Method for producing cell aggregate(s)>

The cell aggregate(s) of the present invention can be produced by the method which comprises a step of providing a base film for cell culture comprising a block copolymer having unit structures represented by the formula (1) and the formula (2): 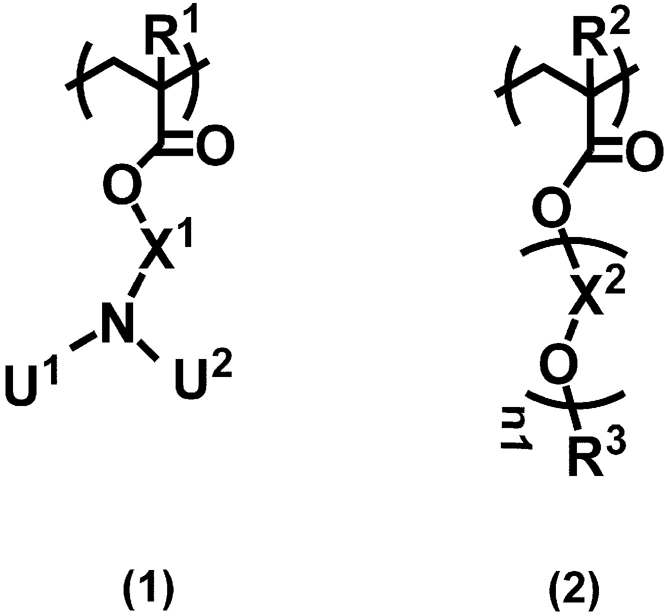 (wherein R¹ to R³, U¹ and U² each independently represent a hydrogen atom or an alkyl group having 1 to 5 carbon atoms, X¹ and X² each independently represent an alkylene group having 1 to 5 carbon atoms, and n1 represents an integer of 1 to 10) on a substrate having an ability to suppress adhesion of a biological substance at least a part of a surface of the substrate, and then, a step of seeding cells on the above-mentioned base film. Production of the cell aggregate(s) can be carried out by a known method and, for example, by the method described in the following Examples.

### EXAMPLES

Hereinafter, the present invention will be explained more specifically by referring to Examples and Comparative Examples, but the present invention is not limited to the following Examples.

The weight average molecular weight (Mw) of the polymer (A) shown in the following Synthetic Example is a measurement result by a gel permeation chromatography (Gel Permeation Chromatography, GPC) method. The measurement conditions are as follows.
GPC column: PL gel 3 µm MIXED-E (manufactured by Agilent Technologies)
Column temperature: 40°C
Solvent: Tetrahydrofuran (THF)
Flow rate: 1.00 ml/ min
Detector: RI detector
Standard sample: Polystyrene
[Synthesis of block copolymer]

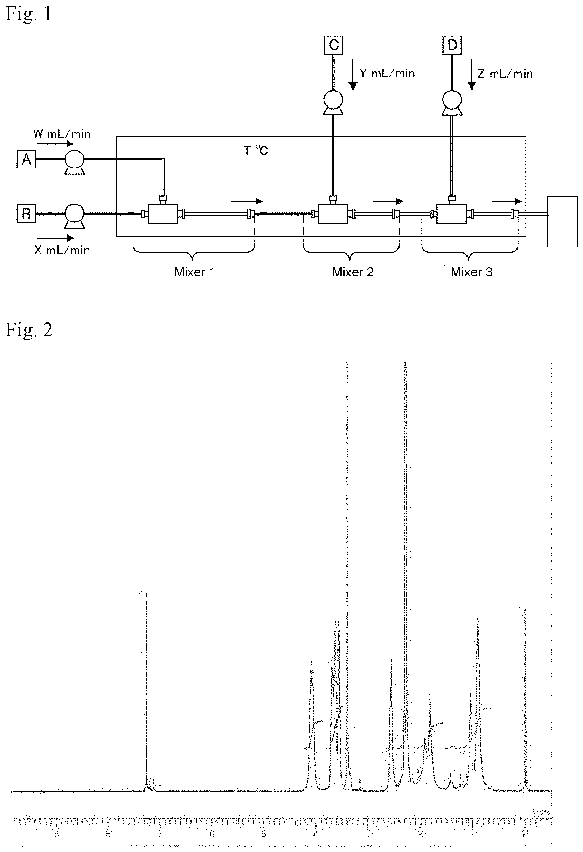

A schematic drawing of the flow reactor (reaction device) used in the following Synthetic Examples 1 to 5 is shown in Fig. 1. Incidentally, in Fig. 1, the arrow indicates the direction in which the liquid flows. A plunger pump A (UI-22-110 manufactured by FLOM Inc.) was used for feeding a first monomer liquid, the plunger pump A and a mixer 1 were connected using a tube made of PTFE (inner diameter 1.0 mm, outer diameter 1.6 mm, length 2 m), a syringe pump B (Keychem-L manufactured by YMC CO., LTD.) was used for feeding an initiator solution, and the syringe pump B and the mixer 1 were connected using a tube made of PTFE (inner diameter 1.0 mm, outer diameter 1.6 mm, length 2 m). The outlet of the mixer 1 and the inlet of a mixer 2 were connected by a tube made of PFA (inner diameter 2.0 mm, outer diameter 3 mm, length 5 m (Synthetic Examples 1, 2 and 3), inner diameter 2.0 mm, outer diameter 3 mm, length 4.5 m (Synthetic Examples 4 and 5)). Another inlet of the mixer 2 was connected by a syringe pump C (Keychem-L manufactured by YMC CO., LTD.) for feeding a second monomer solution and a tube made of PTFE (inner diameter 1.0 mm, outer diameter 1.6 mm, length 2 m). The outlet of the mixer 2 and the inlet of a mixer 3 were connected by a tube made of PFA (inner diameter 2.0 mm, outer diameter 3 mm, length 4 m (Synthetic Examples 1, 2 and 3), inner diameter 2.0 mm, outer diameter 3 mm, length 5 m (Synthetic Examples 4 and 5)). Another inlet of the mixer 3 was connected by a syringe pump D (UI-22-110 manufactured by FLOM Inc.) for feeding a polymerization terminator solution and a tube made of PTFE (inner diameter 1.0 mm, outer diameter 1.6 mm, length 2 m). A tube made of PFA (inner diameter 2.0 mm, outer diameter 3 mm, length 0.7 m) was connected to the outlet of the mixer 3. Incidentally, from each pump to the forward and 90% of the length of the tube connected to the outlet of the mixer 3 was immersed in a thermostatic bath at -40°C to adjust the temperature. As the mixer 1 used for the synthesis, a mixer for two-liquid mixing having a double-tube structure described in WO 2017/135398 [a material made of stainless is used for a joint member and a tubular body, and as a static mixer element body, DSP-MXA3-17 (element made of polyacetal, number of twisted blades 17, 3 mm diameter) manufactured by Noritake Company Limited is processed and three thereof are connected to make the number of twisted blades 51] was used, as the mixer 2, a mixer for two-liquid mixing having a double-tube structure described in the same WO 2017/135398 [a material made of stainless is used for a joint member and a tubular body, and as a static mixer element body, DSP-MXA3-17 (element made of polyacetal, number of twisted blades 17, 3 mm diameter) manufactured by Noritake Company Limited is processed] was used, and as the mixer 3, a general simple double-tube mixer was used. Incidentally, as for the connection method to each mixer, the first monomer solution tube was connected to the inlet of the introduction hole of the mixer 1 and the initiator solution tube was connected to the inlet of the inner tube. The tube connected to the outlet of the mixer 1 was connected to the inlet of the introduction hole of the mixer 2 and the second monomer liquid tube was connected to the inlet of the inner tube. The polymerization terminator solution was connected to the inlet of the introduction hole of the mixer 3 and a tube connected to the outlet of the mixer 2 was connected to the inlet of the inner tube.

### <Synthetic Example 1 Synthesis of poly(2-dimethylaminoethyl methacrylate)-b-poly(diethylene glycol monomethyl ether methacrylate) block polymer (PDM-b-PDEGMA)>

In a mixer 1, a 0.5 mol/L 2-dimethylaminoethyl methacrylate THF solution as a first monomer and a 0.05 mol/L 1,1-diphenylhexyl lithium solution as an initiator were mixed with flow rates of 10 mL/min and 1.5 mL/min, respectively, to polymerize the first monomer. Subsequently, in a mixer 2, a diethylene glycol monomethyl ether methacrylate solution was mixed therewith at 0.92 mL/min as a second monomer to conduct block polymerization. Subsequently, a 0.25 mol/L methanol/THF solution was mixed therewith at 10 mL/min as a polymerization terminator in a mixer 3 to stop the polymerization. Each pump was fed for 5 minutes to collect the flowed liquid.

Further, the solvent was substantially distilled off from the above-mentioned flowed liquid by an evaporator, and then it was added dropwise to a mixed liquid of 200 ml of n-hexane and 200 ml of diethyl ether under an ice water bath. The obtained white suspension was filtered through a 0.5 µm membrane filter. Subsequently, the obtained filtrated product was dissolved in 1,4-dioxane and then freeze-dried to obtain 4.1 g of PDM-b-PDEGMA. When the obtained polymer was analyzed by GPC, it was Mn=29,790 and Mw/Mn=1.19. Also, from the result of ¹H-NMR of the polymer, a compositional ratio of the 2-dimethylaminoethyl methacrylate unit and the diethylene glycol monomethyl ether methacrylate unit was 2-dimethylaminoethyl methacrylate unit: diethylene glycol monomethyl ether methacrylate unit = 48 : 52. The ¹H-NMR chart of the obtained polymer is shown in Fig. 2.

### <Synthetic Example 2 Synthesis of poly(2-dimethylaminoethyl methacrylate)-b-poly(diethylene glycol monomethyl ether methacrylate) block polymer (PDM-b-PDEGMA)>

In the same method as in Synthetic Example 1, synthesis was carried out except for mixing a diethylene glycol monomethyl ether methacrylate liquid as a second monomer in the mixer 2 at 0.46 mL/min and each pump was fed for 7 minutes to obtain 3.4 g of PDM-b-PDEGMA. When the obtained polymer was analyzed by GPC, it was Mn=23,481 and Mw/Mn=1.11. Also, from the result of ¹H-NMR of the polymer, a compositional ratio of the 2-dimethylaminoethyl methacrylate unit and the diethylene glycol monomethyl ether methacrylate unit was 2-dimethylaminoethyl methacrylate unit: diethylene glycol monomethyl ether methacrylate unit = 63 : 37.

### <Synthetic Example 3 Synthesis of poly(2-dimethylaminoethyl methacrylate)-b-poly(diethylene glycol monomethyl ether methacrylate) block polymer (PDM-b-PDEGMA)>

In the same method as in Synthetic Example 1, synthesis was carried out except for mixing a diethylene glycol monomethyl ether methacrylate liquid as a second monomer in the mixer 2 at 0.19 mL/min and each pump was fed for 8 minutes to obtain 4.4 g of PDM-b-PDEGMA. When the obtained polymer was analyzed by GPC, it was Mn=15,065 and Mw/Mn=1.17. Also, from the result of ¹H-NMR of the polymer, a compositional ratio of the 2-dimethylaminoethyl methacrylate unit and the diethylene glycol monomethyl ether methacrylate unit was 2-dimethylaminoethyl methacrylate unit: diethylene glycol monomethyl ether methacrylate unit = 88 : 12.

### <Synthetic Example 4 Synthesis of poly(2-diethylaminoethyl methacrylate)-b-poly(diethylene glycol monomethyl ether methacrylate) block polymer (PDE-b-PDEGMA)>

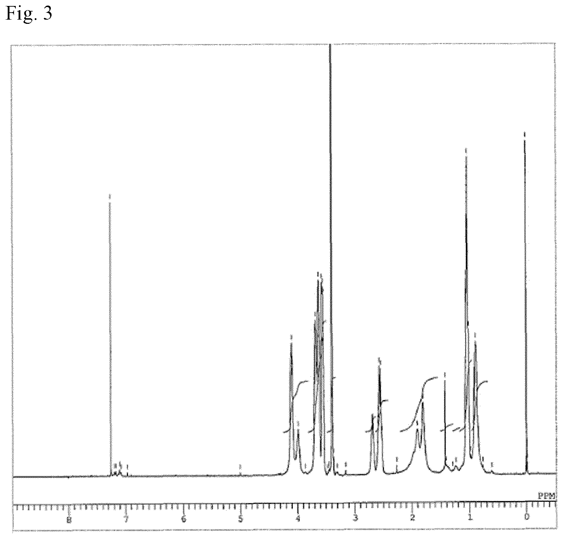

In the same method as in Synthetic Example 1, synthesis was carried out except for mixing a 0.5 mol/L 2-diethylaminoethyl methacrylate THF solution as the first monomer and a 0.10 mol/L 1,1-diphenylhexyl lithium solution as the initiator in the mixer 1 with flow rates of 10 mL/min and 1.5 mL/min, respectively, and mixing a diethylene glycol monomethyl ether methacrylate liquid as the second monomer in the mixer 2 at 0.92 mL/min, and each pump was fed for 6 minutes to obtain 2.5 g of PDE-b-PDEGMA. When the obtained polymer was analyzed by GPC, it was Mn=17,555 and Mw/Mn=1.21. Also, from the result of ¹H-NMR of the polymer, a compositional ratio of the 2-diethylaminoethyl methacrylate unit and the diethylene glycol monomethyl ether methacrylate unit was 2-diethylaminoethyl methacrylate unit : diethylene glycol monomethyl ether methacrylate unit = 29 : 71. The ¹H-NMR chart of the obtained polymer is shown in Fig. 3.

### <Synthetic Example 5 Synthesis of poly(2-diethylaminoethyl methacrylate)-b-poly(diethylene glycol monomethyl ether methacrylate) block polymer (PDE-b-PDEGMA)>

In the same method as in Synthetic Example 4, synthesis was carried out except for mixing a 0.5 mol/L 2-diethylaminoethyl methacrylate THF solution as the first monomer and a 0.10 mol/L 1,1-diphenylhexyl lithium solution as the initiator in the mixer 1 with flow rates of 10 mL/min and 2.0 mL/min, respectively, and mixing a diethylene glycol monomethyl ether methacrylate liquid as the second monomer in the mixer 2 at 1.48 mL/min to obtain 1.6 g of PDE-b-PDEGMA. When the obtained polymer was analyzed by GPC, it was Mn=16,488 and Mw/Mn=1.21. Also, from the result of ¹H-NMR of the polymer, a compositional ratio of the 2-diethylaminoethyl methacrylate unit and the diethylene glycol monomethyl ether methacrylate unit was 2-diethylaminoethyl methacrylate unit: diethylene glycol monomethyl ether methacrylate unit = 43 : 57.

### <Comparative Synthetic Example 1 Synthesis of poly(2-dimethylaminoethyl methacrylate)-r-poly(diethylene glycol monomethyl ether methacrylate) random polymer (PDM-r-PDEGMA)>

To 1.0 g of 2-dimethylaminoethyl methacrylate and 1.0 g of diethylene glycol monomethyl ether methacrylate were added 18.0 g of THF and 11.1 mg of 2,2'-azodiisobutyronitrile, and dissolved. Thereafter, under nitrogen atmosphere, the temperature of the water bath was set to 60°C, and the mixture was stirred for 18 hours. After the temperature of the reaction mixture was returned to room temperature, the mixture was added dropwise to 500 mL of hexane, and the obtained white suspension was filtered through a 0.5 µm membrane filter. Subsequently, the obtained filtered product was dissolved in water and freeze-dried to obtain 1.7 g of PDM-r-PDEGMA. When the obtained polymer was analyzed by GPC, it was Mn=12,317 and Mw/Mn=2.52 Also, from the result of ¹H-NMR of the polymer, a compositional ratio of the 2-dimethylaminoethyl methacrylate unit and the diethylene glycol monomethyl ether methacrylate unit was 2-dimethylaminoethyl methacrylate unit : diethylene glycol monomethyl ether methacrylate unit = 46 : 54.

### <Comparative Synthetic Example 2 Synthesis of poly(2-diethylaminoethyl methacrylate)-r-poly(diethylene glycol monomethyl ether methacrylate) random polymer (PDE-r-PDEGMA)>

To 1.0 g of 2-diethylaminoethyl methacrylate and 1.4 g of diethylene glycol monomethyl ether methacrylate were added 21.2 g of THF and 11.8 mg of 2,2'-azodiisobutyronitrile, and dissolved. Thereafter, under nitrogen atmosphere, the temperature of the water bath was set to 60°C, and the mixture was stirred for 21.5 hours. After the temperature of the reaction mixture was returned to room temperature, the mixture was added dropwise to 300 mL of hexane, and the obtained white suspension was filtered through a 0.5 µm membrane filter. Subsequently, the obtained filtered product was dissolved in 1,4-dioxane and freeze-dried to obtain 1.8 g of PDE-r-PDEGMA.

When the obtained polymer was analyzed by GPC, it was Mn=18,107 and Mw/Mn=2.31. Also, from the result of ¹H-NMR of the polymer, a compositional ratio of the 2-diethylaminoethyl methacrylate unit and the diethylene glycol monomethyl ether methacrylate unit was 2-diethylaminoethyl methacrylate unit : diethylene glycol monomethyl ether methacrylate unit = 46 : 54.

### <Comparative Synthetic Example 3 Synthesis of poly(2-diethylaminoethyl methacrylate)-r-poly(methacrylic acid) random polymer (PDM-r-PMA)>

In accordance with the producing method described in Synthetic Example 8 of WO 2020/040247, PDM-r-PMA was obtained. When the obtained polymer was analyzed by GPC, it was Mn=472,133 and Mw/Mn=3.73.

### <Preparation Examples 1 to 5 and Comparative Preparation Examples 1 to 3: Preparation of polymer ethanol solution>

The polymers of Synthetic Examples 1 to 5 and Comparative Synthetic Examples 1 to 3 were each dissolved in ethanol so that it became a concentration of 10 mg/mL to prepare each composition for forming a coating film (Preparation Examples 1 to 5 and Comparative Preparation Examples 1 to 3).

### <Preparation Example 6 and Comparative Preparation Example 4: Preparation of polymer aqueous solution>

The polymers of Synthetic Example 1 and Comparative Synthetic Example 1 were each dissolved in sterilized water to have a concentration of 1 mg/mL to prepare a composition for forming a coating film (Preparation Example 6 and Comparative Preparation Example 4).

### <Preparation Example 7: Preparation of polymer aqueous solution>

The polymer of Synthetic Example 1 was dissolved in sterilized water so as to be a concentration of 5 mg/mL to prepare a composition for forming a coating film (Preparation Example 7).

### <Preparation Example 8, Comparative Preparation Example 5: Preparation of polymer solution>

The polymer of Synthetic Example 5 and Comparative Synthetic Example 2 were each dissolved in a mixed solution of sterilized water/ethanol=7/3 so as to be a concentration of 1 mg/mL to prepare a composition for forming a coating film (Preparation Example 8 and Comparative Preparation Example 5).

### <Preparation Example 9: Preparation of polymer solution>

The polymer of Synthetic Example 5 was dissolved in sterilized a mixed solution of water/ethanol=7/3 so as to be a concentration of 5 mg/mL to prepare a composition for forming a coating film (Preparation Example 9).

### <Test Example 1: Coating film formation test>

The compositions for forming a coating film obtained in the above-mentioned Preparation Example 1 to 5 and Comparative Preparation Examples 1 to 3 were each spin-coated on an HMDS-treated silicon wafer with 1,500 rpm for 60 sec, and, as a drying step, dried in an oven at 70°C for 24 hours. Thereafter, after thoroughly washing with PBS, it was dried in an oven at 70°C for 1 hour to obtain a coating film on the HMDS-treated silicon wafer. A film thickness of the coating film on the HMDS-treated silicon wafer measured by using a spectroscopic ellipsometer is shown in the following Table 1. A coating film was formed when any of the compositions for forming a coating film was used.

**[Table 1]**

| Polymer | Coating agent | Film thickness [nm] |
|---|---|---|
| Synthetic Example 1 | Preparation Example 1 | 44.7 |
| Synthetic Example 2 | Preparation Example 2 | 42.6 |
| Synthetic Example 3 | Preparation Example 3 | 38.1 |
| Synthetic Example 4 | Preparation Example 4 | 20.0 |
| Synthetic Example 5 | Preparation Example 5 | 32.9 |
| Comparative Synthetic Example 1 | Comparative Preparation Example 1 | 38.1 |
| Comparative Synthetic Example 2 | Comparative Preparation Example 2 | 36.6 |
| Comparative Synthetic Example 3 | Comparative Preparation Example 3 | 72.3 |
| - | None | 1.9 |

### <Test Example 2: Protein adhesion suppressing test>

### (Preparation of QCM sensor (PS))

An Au-deposited crystal oscillator (Q-Sence, QSX301) was washed using a soft etching device (SEDE-GE manufactured by MEIWAFOSIS CO., LTD.) with 50 mA/3 min, and immediately thereafter, it was immersed in a solution in which 0.0772 g of 2-aminoethanethiol (available from Tokyo Chemical Industry Co., Ltd.) was dissolved in 1,000 mL of ethanol for 24 hours. After washing the surface of the sensor with ethanol, it was naturally dried, and a varnish in which 1.00 g of polystyrene (PS) (available from Sigma-Aldrich) was dissolved in 9.00 g of toluene was spin-coated onto a sensor side of the film at 3,500 rpm for 30 sec by a spin coater and dried at 150°C/1 min to prepare a QCM sensor (PS).

### (Preparation of QCM sensor (SiO₂))

An SiO₂-deposited crystal oscillator (Q-Sence, QSX303) was washed using a soft etching device (SEDE-GE manufactured by MEIWAFOSIS CO., LTD.) with 50 mA/3 min, and used as such.

### (Preparation of surface-treated QCM sensor (PS) and surface-treated QCM sensor (SiO₂))

Each composition for forming a coating film obtained in Preparation Examples 1 to 5 and Comparative Preparation Example 1 was spin-coated on the QCM sensor (PS) at 3,500 rpm for 30 sec, and as a drying step, it was baked in an oven at 70°C for 24 hours. Thereafter, as a washing step, the excessively attached composition for forming a coating film was washed with PBS and pure water each twice to obtain a surface-treated QCM sensor (PS) (Substrates No. 1 to 6). Similarly, each composition for forming a coating film obtained in Preparation Examples 1 to 5, Comparative Preparation Examples 1 to 2 was spin-coated on a QCM sensor (SiO₂) at 3,500 rpm for 30 sec, and as a drying step, it was baked with a hot plate at 100°C for 5 hours. Thereafter, as a washing step, the excessively attached composition for forming a coating film was washed with PBS and pure water each twice to obtain a surface-treated QCM sensor (SiO₂) (Substrates Nos. 8 to 14). As a reference, a QCM sensor (PS) (Substrate No. 7) and QCM sensor (SiO₂) (Substrate No. 15) were used.

### (Protein adhesion test)

Using the composition for forming a coating film, the surface-treated QCM sensor (PS) (Substrates Nos. 1 to 7) and surface-treated QCM sensor (SiO₂) (the substrate Nos. 8 to 15) prepared by the method as mentioned above was attached to a dissipation type quartz crystal microbalance QCM-D (E4, Q-Sence), and PBS was flowed until a stable baseline was established in which change in the frequency became 1 Hz or less in 1 hour. Next, by making the frequency of the stable baseline as 0 Hz, PBS was flowed for about 10 minutes. Subsequently, a 100 µg/mL PBS solution of human serum-derived γ-globulin was flowed for about 30 minutes, and thereafter, the shift (Δf) of the adsorption-induced frequency of the 9th-order overtone was read after PBS was flowed again for about 20 minutes. Using Q-Tools (Q-Sense, manufactured by Biolin Scientific) for analysis, the value converted the shift (Δf) of the adsorption-induced frequency into a mass (ng/cm²) per a unit surface area of the shift (Δf) of the adsorption-induced frequency explained by the Sauerbrey equation is shown in the following Table 2 as an adhered amount of the biological substance. The substrates Nos. 1 to 5 and the substrates Nos. 8 to 12 using the compositions for forming a coating film of Preparation Examples 1 to 5 according to the present invention as a coating showed markedly low protein adhered amount as compared with the substrates Nos.7 and 15 having no coating film. In addition, when they are compared with the substrate No. 6 and the substrates Nos. 13 to 14 using the compositions for forming a coating film prepared in Comparative Preparation Examples 1 to 2, it was shown that adhesion of proteins could be dominantly suppressed.

**Table 2]**

| Substrate No. | QCM Sensor | Polymer | Coating agent | Protein adhered amount (ng/cm²) |
|---|---|---|---|---|
| 1 | PS | Synthetic Example 1 | Preparation Example 1 | 7 |
| 2 | PS | Synthetic Example 2 | Preparation Example 2 | 62 |
| 3 | PS | Synthetic Example 3 | Preparation Example 3 | 20 |
| 4 | PS | Synthetic Example 4 | Preparation Example 4 | 14 |
| 5 | PS | Synthetic Example 5 | Preparation Example 5 | 15 |
| 6 | PS | Comparative Synthetic Example 1 | Comparative Preparation Example 1 | 159 |
| 7 | PS | - | None | 1335 |
| 8 | SiO₂ | Synthetic Example 1 | Preparation Example 1 | 22 |
| 9 | SiO₂ | Synthetic Example 2 | Preparation Example 2 | 54 |
| 10 | SiO₂ | Synthetic Example 3 | Preparation Example 3 | 94 |
| 11 | SiO₂ | Synthetic Example 4 | Preparation Example 4 | 5 |
| 12 | SiO₂ | Synthetic Example 5 | Preparation Example 5 | 25 |
| 13 | SiO₂ | Comparative Synthetic Example 1 | Comparative Preparation Example 1 | 324 |
| 14 | SiO₂ | Comparative Synthetic Example 2 | Comparative Preparation Example 2 | 73 |
| 15 | SiO₂ | - | None | 941 |

### <Test Example 3: Adhesion suppressing test of protein, etc., in medium>

### (Preparation of surface-treated QCM sensor (PS))

In the same manner as in Test Example 2, after preparing the QCM sensor (PS), each composition for forming a coating film obtained in Preparation Examples 1 to 5 and Comparative Preparation Examples 3 was spin-coated at 3,500 rpm for 30 sec on the QCM sensor (PS), and as a drying step, it was baked in an oven at 70°C for 24 hours. Thereafter, as a washing step, the excessively attached composition for forming a coating film was washed with PBS and pure water each twice to obtain a surface-treated QCM sensor (PS) (Base materials Nos. 1 to 5 and 16).

### (Adhesion experiment of protein, etc., in medium)

Using the composition for forming a coating film, the surface-treated QCM sensor (PS) (Substrates Nos. 1 to 5 and 16) prepared by the method as mentioned above was attached to a dissipation type quartz crystal microbalance QCM-D (E4, Q-Sence), and PBS was flowed until a stable baseline was established in which change in the frequency became 1 Hz or less in 1 hour. Next, by making the frequency of the stable baseline as 0 Hz, PBS was flowed for about 10 minutes. Subsequently, a BME medium (available from Thermo Fisher Scientific K. K.) containing a 10% FBS (available from Sigma-Aldrich) and a 1% L-glutamine-penicillin-streptomycin stabilized solution (available from Thermo Fisher Scientific K. K.) was flowed for about 30 minutes, and the shift (Δf) of the adsorption-induced frequency of the 9th-order overtone was read. Using Q-Tools (Q-Sence) for analysis, the value converted the shift (Δf) of the adsorption-induced frequency into a mass (ng/cm²) per a unit surface area of the shift (Δf) of the adsorption-induced frequency explained by the Sauerbrey equation is shown in the following Table 3 as an adhered amount of the biological substance. The substrates Nos. 1 to 5 using the compositions for forming a coating film of Preparation Examples 1 to 5 according to the present invention as a coating showed a low adhered amount of protein, etc., as compared with the substrate No. 16 using the composition for forming a coating film of Comparative Preparation Example 3 as a coating.

**[Table 3]**

| Substrate No. | QCM Sensor | Polymer | Coating agent | Protein adhered amount (ng/cm²) |
|---|---|---|---|---|
| 1 | PS | Synthetic Example 1 | Preparation Example 1 | 3039 |
| 2 | PS | Synthetic Example 2 | Preparation Example 2 | 6414 |
| 3 | PS | Synthetic Example 3 | Preparation Example 3 | 3430 |
| 4 | PS | Synthetic Example 4 | Preparation Example 4 | 113 |
| 5 | PS | Synthetic Example 5 | Preparation Example 5 | 220 |
| 16 | PS | Comparative Synthetic Example 3 | Comparative Preparation Example 3 | 11720 |

### <Test Example 4: Formation test of cell aggregates>

### (Preparation of cell-low-adhesion petri dish)

According to the producing method described in Example 30 of WO 2014/196650, a coating solution was prepared from a copolymer-containing varnish. The prepared coating solution was added to ϕ40 mm ASNOL petri dish (#1-8549-01 manufactured by AS ONE Corporation) with each 1 mL and after allowing to stand at room temperature for 1 hour, excess coating solution was removed and it was baked in an oven at 50°C for 24 hours. Thereafter, 2 mL of sterilized water was added thereto and then the liquid was discarded to carry out washing. The same washing operation was further carried out twice, and it was dried in an oven at 50°C for 1 hour to obtain a cell low-adhesion petri dish.

### (Preparation of petri dish for forming cell aggregates by inkjet)

Using an inkjet device (Model number: LaboJet-600 manufactured by MICROJET Corporation) and an inkjet head (Model number: 500-S-C), the compositions for forming a coating film prepared in Preparation Examples 6 to 9 and Comparative Preparation Examples 4 and 5 were each coated in a spot shape (circular shape) to the cell low-adhesion petri dish prepared as mentioned above with each about 50 nL. The dish was dried in an oven at 70°C for 24 hours to prepare a petri dish (petri dishes Nos. 1 to 6) for forming cell aggregates.

### (Preparation of cells)

As cells, mouse embryo fibroblasts C3H10T1/2 (available from DS Pharma Biomedical) were used. As a medium used for culture of cells, a BME medium (available from Thermo Fisher Scientific K. K.) containing a 10% FBS (available from Sigma-Aldrich) and an L-glutamine-penicillin-streptomycin stabilized solution (available from Thermo Fisher Scientific K. K.) was used. The cells were statically cultured in a petri dish (medium 10 mL) having a diameter of 10 cm for 2 days or longer while maintaining a 5% carbon dioxide concentration in a 37°C/CO₂ incubator. Subsequently, after washing the present cells with 5 mL of PBS, 1 mL of 0.25 w/v% trypsin-1 mmol/L EDTA solution (available from FUJIFILM Wako Pure Chemical Corporation) was added thereto to peel off the cells and the cells were suspended in 10 mL of the above-mentioned medium, respectively. After the present suspension was centrifuged (Model number LC-200 manufactured by TOMY SEIKO Co., Ltd., 1,000 rpm for 3 min, room temperature), the supernatant was removed and the above-mentioned medium was added thereto to prepare a cell suspension.

### (Observation of cell adhesion)

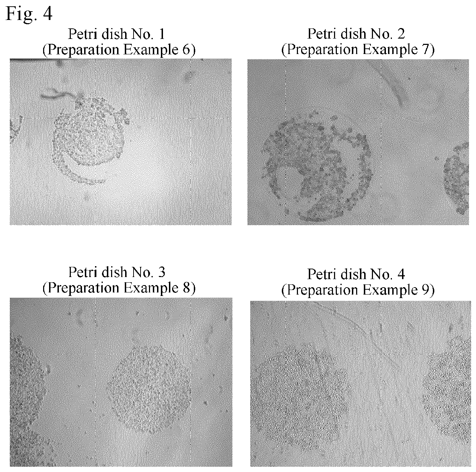

Each 2 mL of cell suspension was added to the petri dish for forming cell aggregates prepared as mentioned above so as to be 6.0×10⁵ cells/cm². Thereafter, in the state maintaining a 5% carbon dioxide concentration, it was allowed to stand in a CO₂ incubator at 37°C for 2 hours. After allowing to stand, using an inverted microscope (ECLIPSE TS100-F manufactured by Nikon Corporation), it was confirmed that the cells adhered to the spots where each coating agent was spotted. Thereafter, the non-adherent cells and the medium were removed by an aspirator and washed with PBS to leave only the adhered cells on the wells. After washing, each 2 mL of new medium was added, and the state of the adhered cells was observed and photographed using an inverted research microscope IX73 (manufactured by Olympus Corporation). As a result, as shown in Fig. 4, it was confirmed that adhesion of the cells to the spotted portion for forming the coating film of the petri dishes Nos. 1 to 4 at which the compositions for forming a coating film prepared in Preparation Examples 6 to 9 had been coated in a spotted state was maintained. On the other hand, it was confirmed that the cells adhered to the spotted portion for forming the coating film of the petri dishes Nos. 5 and 6 at which the compositions for forming a coating film prepared in Comparative Preparation Examples 4 and 5 had been coated in a spotted state were removed by washing.

### (Observation of cell aggregates)

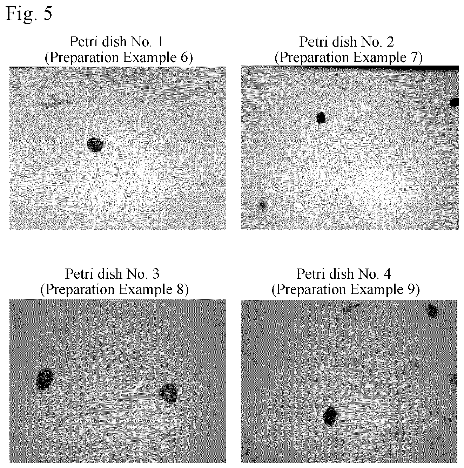

The petri dish whose cell adhesion was confirmed as mentioned above was allowed to stand in a CO₂ incubator at 37°C for further 2 days. After allowing to stand, the state of the cells was observed using an inverted research microscope IX73 (manufactured by Olympus Corporation). As a result, as shown in Fig. 5, it was confirmed that the cells which had been adhered to the spotted portion for forming the coating film of the petri dishes Nos. 1 to 4 at which the compositions for forming a coating film prepared in Preparation Examples 6 to 9 had been coated in a spotted state were peeled off from the plate and aggregated to form a cell aggregate (spheroid). From this results, it was suggested that the base film containing the polymer of the present invention is useful as a base film of a cell culture container. The results at each stage of cell adhesion before washing, maintenance of cell adhesion after washing, and formation of cell aggregates are shown in Table 4.

**Table 4]**

| Petri dish No. | Polymer | Coating agent | Cell adhesion before washing | Cell adhesion after washing | Cell aggregates formation |
|---|---|---|---|---|---|
| 1 | Synthetic Example 1 | Preparation Example 6 | ○ | ○ | ○ |
| 2 | Synthetic Example 1 | Preparation Example 7 | ○ | ○ | ○ |
| 3 | Synthetic Example 5 | Preparation Example 8 | ○ | ○ | ○ |
| 4 | Synthetic Example 5 | Preparation Example 9 | ○ | ○ | ○ |
| 5 | Comparative Synthetic Example 1 | Comparative Preparation Example 4 | ○ | × | - |
| 6 | Comparative Synthetic Example 2 | Comparative Preparation Example 5 | ○ | × | - |

### UTILIZABILITY IN INDUSTRY

According to the present invention, it is possible to provide a composition for forming a coating film having compatibility with a biological substance, a coating film using the same, and a substrate for cell culture using the same.

## Claims

1. A composition for forming a coating film having compatibility with a biological substance which comprises a block copolymer having unit structures represented by the formula (1) and the formula (2): 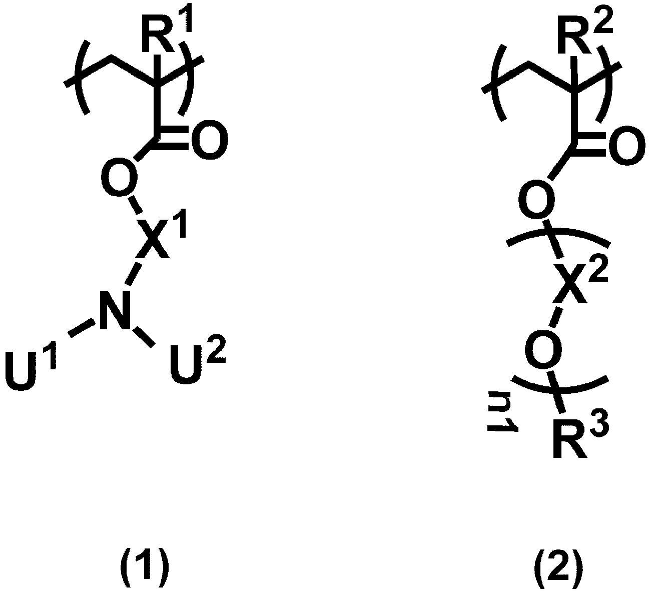 wherein R¹ to R³, U¹ and U² each independently represent a hydrogen atom or an alkyl group having 1 to 5 carbon atoms, X¹ and X² each independently represent an alkylene group having 1 to 5 carbon atoms, and n1 represents an integer of 1 to 10,
and a solvent.

2. The composition according to Claim 1, wherein the solvent contains water or an alcohol.

3. The composition according to Claim 1 or 2, wherein the compatibility with a biological substance is an ability to suppress adhesion of a protein.

4. The composition according to Claim 1 or 2, wherein the compatibility with a biological substance is for forming a base film for cell culture.

5. The composition according to Claim 4, which is used for forming a base film for cell culture to obtain a cell aggregate by adhering and then peeling cells.

6. A coating film having compatibility with a biological substance, which is a coating film of the composition for forming a coating film having compatibility with a biological substance according to any one of Claims 1 to 5.

7. A substrate for cell culture, which comprises the coating film having compatibility with a biological substance according to Claim 6 at least at a part of a surface of a substrate having an ability to suppress adhesion of a biological substance.

8. The substrate for cell culture according to Claim 7, wherein the substrate having an ability to suppress adhesion of a biological substance is a substrate provided with a coating film containing a copolymer which contains a recurring unit containing a group represented by the following formula (a) and a recurring unit containing a group represented by the following formula (b): 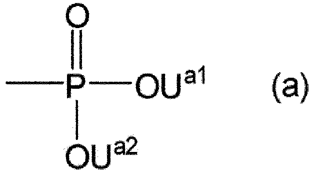 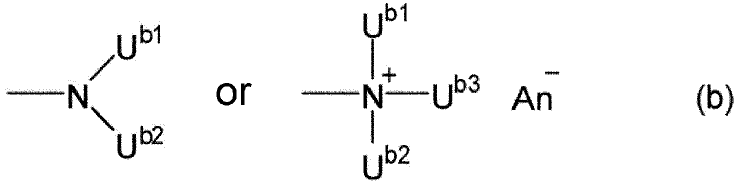 wherein
U^{a1}, U^{a2}, U^{b1}, U^{b2} and U^{b3} each independently represent a hydrogen atom or an alkyl group having 1 to 5 carbon atoms, An⁻ represents an anion selected from the group consisting of a halide ion, an inorganic acid ion, a hydroxide ion and an isothiocyanate ion
on at least a part of a surface thereof.

9. A method for producing a substrate for cell culture which comprises a step of applying a composition for forming a coating film having compatibility with a biological substance containing a block copolymer having unit structures represented by the formula (1) and the formula (2): 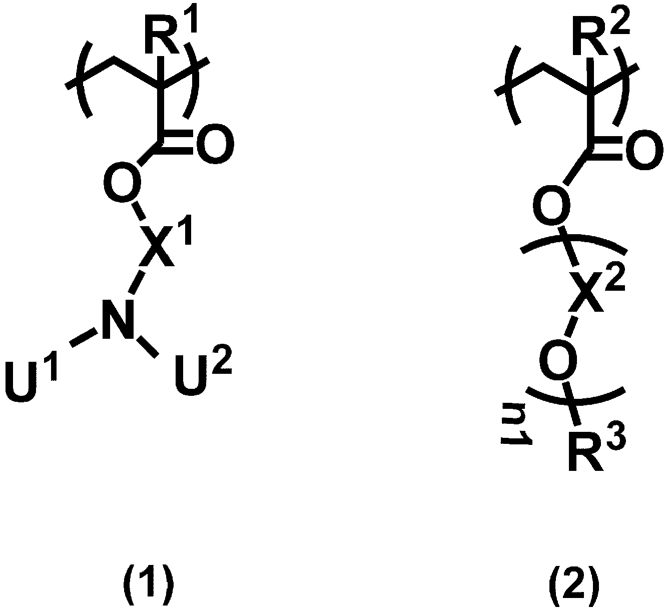 wherein R¹ to R³, U¹ and U² each independently represent a hydrogen atom or an alkyl group having 1 to 5 carbon atoms, X¹ and X² each independently represent an alkylene group having 1 to 5 carbon atoms, and n1 represents an integer of 1 to 10,
and a solvent to a substrate having an ability to suppress adhesion of a biological substance, and then, a step of drying the same.

10. A method for producing a cell aggregate which comprises a step of providing a base film for cell culture comprising a block copolymer having unit structures represented by the formula (1) and the formula (2): 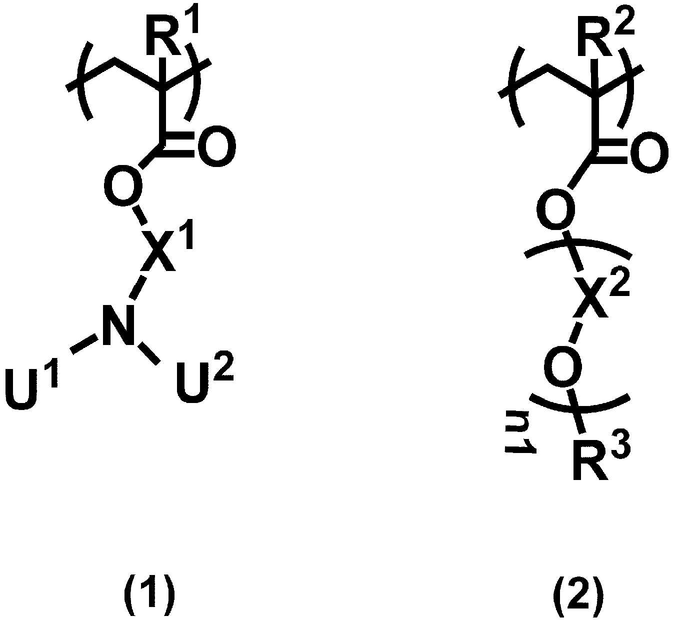 wherein R¹ to R³, U¹ and U² each independently represent a hydrogen atom or an alkyl group having 1 to 5 carbon atoms, X¹ and X² each independently represent an alkylene group having 1 to 5 carbon atoms, and n1 represents an integer of 1 to 10,
onto at least a part of a surface of a substrate, and a step of seeding cells on the base film.
